(19) Europäisches Patentamt
European Patent Office
Office européen des brevets

(11) Publication number: 0 231 546 B1

# (12) EUROPEAN PATENT SPECIFICATION

(45) Date of publication of the patent specification: 07.11.90

(51) Int. Cl.⁵: C07C 231/06

(21) Application number: 86202260.5

(22) Date of filing: 15.12.86

## (54) Process for the preparation of alpha-amino-alpha-methylcarboxylic acid amides and alpha-amino-alpha-cycloalkylcarboxylic acid amides.

(30) Priority: 22.01.86 NL 8600131

(43) Date of publication of application: 12.08.87 Bulletin 87/33

(45) Publication of the grant of the patent: 07.11.90 Bulletin 90/45

(84) Designated Contracting States: AT BE CH DE ES FR GB IT LI NL SE

(56) References cited:
CHEMICAL ABSTRACTS, vol. 102, no. 13, 1st April 1985, page 671, abstract no. 113066u, Columbus, Ohio, US; & JP-A-59 161 341 (UBE INDUSTRIES, LTD) 12-09-1984

(73) Proprietor: STAMICARBON B.V., Mijnweg 1, NL-6167 AC Geleen(NL)

(72) Inventor: Boesten, Wilhelmus H. J., Brountslaan 9, NL-6132 BJ Sittard(NL)
Inventor: Kamphuis, Johan, Aureliushof 129h, NL-6215 SR Maastricht(NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of α-amino-α-methylcarboxylic acid amides and α-amino-α-cycloalkylcarboxylic acid amides having the formula:

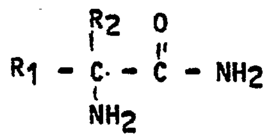

where $R_1$ represents alkyl, alkaryl, aryl, alkenyl, heteroalkyl or hetero-aryl, whether or not substituted, and $R_2$ is methyl, or forms, together with $R_1$, a cycloalkyl, by conversion of the corresponding amino nitriles using hydrogen peroxide under basic conditions.

The α-amino-α-alkylcarboxylic acid amides can be applied as intermediary in the synthesis of optically pure α-methyl amino acids. Both the amides and the acids can be applied as intermediaries in the pharmaceutical, the agricultural and the flavours and fragrances industries.

JP-A-83-31830 discloses the preparation of 3-phenyl-2-amino-2-methylpropionic acid amides by contacting a corresponding nitrile under basic conditions with hydrogen peroxide. By preference 10-60 moles hydrogen peroxide are used per mole nitrile, and 2-10 moles base, such as alkali metal hydroxides or carbonates. The reaction is preferably carried out between 0 and 5 °C during 4 hours.

The drawback of such a process resides notably in the application of a large excess of hydrogen peroxide and alkali metal hydroxide or carbonate. This renders the process relatively expensive. Furthermore, during purification of the amide obtained inorganic salts are to be removed.

Salt-free working is necessary notably if the amide obtained is converted into the L-acid and the D-amide using enzymatic methods, the reaction rate of an enzymatic conversion being adversely affected by high salt concentrations.

The object of the invention is elimination of the above-mentioned drawbacks.

The invention therefore provides a process for the preparation of α-amino-α-methylcarboxylic acid amides and α-amino-α-cycloalkylcarboxylic acid amides of the formula:

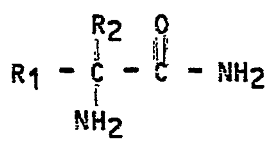

where $R_1$ represents alkyl, alkaryl, aryl, alkenyl or heteroaryl and $R_2$ is methyl or forms, together with $R_1$, a cycloalkyl, by conversion of the corresponding amino nitriles using hydrogen peroxide under basic conditions, and is characterized in that the conversion takes place in the presence of 2–6 moles hydrogen perioxide per mole amino nitrile and in the presence of 3–12 moles ammonia per mole amino nitrile.

Thus, it is achieved that the reaction proceeds exceedingly well with an amount of hydrogen peroxide that is small relative to JP-A 8 331 380.

A great advantage is that no inorganic salts need to be removed during purification of the amide obtained, because the ammonia can, after reduction of the excess hydrogen peroxide with, for instance, palladium-on-carbon, simply be removed by distillation.

Another advantage is that the α-amino-α-alkylcarboxylic acid amides obtained can simply be converted into the corresponding α-amino acids using 1 to 2 equivalents of a strong base such as NaOH or KOH. It is true that an α-amino acid can be prepared directly from the corresponding α-amino nitrile, but this is possible only if 6 equivalents of a strong acid, such as HCl, are applied. This has the disadvantage that the environment in that case is highly corrosive.

In itself, it is known from GB-B 1 548 032 to convert α-amino nitriles into the corresponding amides by application of a base and a ketone. Besides the disadvantage referred to, the need to remove inorganic salts, this process appears to lead to very long reaction times for sterically hindered α-alkyl-α-amino nitriles, and in most cases the reactions do not proceed at all.

The process according to the invention is preferably carried out in the presence of 2-6 moles hydrogen peroxide, in particular in the presence of about 2.2 moles hydrogen peroxide, per mole α-amino-α-alkyl nitrile. In general it is attempted not to use more moles hydrogen peroxide per mole α-amino-α-alkyl nitrile than is strictly necessary.

The amount of ammonia to be applied in the process according to the invention may vary between 3 and 12 moles $NH_3$ per mole α-amino-α-alkyl nitrile. Use is made in particular of between 5 and 8 moles $NH_3$. The reaction generally proceeds well if the pH remains higher than 9. Upon termination of the reaction the remaining ammonia can, after decomposition of hydrogen peroxide with a reducing agent, be removed

by, for instance, distillation. This prevents the formation, besides the desired α-amino-α-alkylcarboxylic acid amides of salts that must subsequently be removed.

The process according to the invention can be carried out at various temperatures. Preferably a temperature between 0 and 50°C is used, in particular between 25 and 45°C, the amino nitriles being thermally unstable. The allowable temperature is determined by the stability of the α-amino nitrile. The α-amino nitriles decompose notably above 60°C into cyanide and ketones, upon which the cyanide may polymerize under the reaction conditions then prevailing.

As solvents the reaction medium generally contains water, lower alcohols such as methanol, ethanol, propanol, butanol, etc. Higher alcohols can in principle also be used, but are less interesting because of their poorer compatibility with water. Furthermore, other organic solvents that can be mixed with water can be applied, such as dioxane and tetrahydrofuran.

In the way described above the reaction is completed within a few hours.

The reaction is not affected by $R_1$, which may in principle therefore be chosen at random. $R_1$ will generally be an alkyl, alkenyl, aryl, alkaryl, heteroalkyl or heteroaryl group, it of course being possible for the alkyl group to be cyclic. The bulkiness of this group is not important, either, but mostly it will not contain more than 20 carbon atoms. $R_1$ may optionally be a substituted hydrocarbon residue, insofar as the substituent does not react under the reaction conditions. As such notably oxygen and nitrogen containing compounds can be mentioned, in particular alkoxy and alkyl amine. $R_2$ is methyl or forms, together with $R_1$, a cycloalkyl group.

The optically active α-amino-α-alkylcarboxylic acid amides and corresponding acids will generally find application in compounds in which they are active as amino acid analogues. $R_1$ in particular therefore preferably is an organic group, whether or not substituted, that is closely related to corresponding groups of natural amino acids. Examples of these are methyl, ethyl, isopropyl, 2-butyl, phenyl, the tryptophane group (benzopyrrolyl), methoxy-substituted benzyl groups, methylimidazolyl, hydroxymethyl and methoxymethyl.

The α-amino nitriles may for instance by synthesized by addition of hydrocyanic acid to ketones in an ammonia containing environment.

A process for this is described, for instance, in JP-A-57156448 and Org. Synth. Collect. Vol. 3, p. 88 (R.E. Steiger).

Conversion of the amino nitrile into the corresponding amide may take place immediately upon the addition. It is not necessary, and often even undesirable, to isolate the α-amino nitrile since these amino nitriles may be thermally unstable.

After the conversion into α-amino-α-alkylcarboxylic acid amides, which are stable at the usual temperatures, first the excess hydrogen peroxide can be reduced by means of a reducing agent, after which the excess ammonia can simply be evaporated. If desired, subsequently separation of the optical isomers can take place. By such an optical separation of the D,L-amide, for instance by applicaton of an amidase-containing enzyme preparation obtained from a culture of Myco bacterium neoaurum, the L-α-amino acid can be obtained. Separation of the optical isomers could, however, also be effected using physicochemical methods, in which the already virtually pure starting material can very simply be used.

The α-amino-α-alkylcarboxylic acid amides and corresponding acids may serve as intermediaries in the synthesis of products for the pharmaceutical, the agricultural and the flavours and fragrances industries. Mention may be made in particular of the L-α-MeDOPA, used as anti-hypertensive and against Parkinson's disease, and the 1-carboxylic acid-1-aminocyclopentane, which displays antitumor activity.

The invention will be elucidated with reference to the following examples, without being restricted thereto.

Example I

An amount of 30 ml concentrated acetic acid was slowly added to 300 ml concentrated ammonia and 70 ml NaCN (30% solution). The temperature was kept below 40 ¤C. At a temperature ≤ 40 ¤C, 0.5 mole (43 g) 3-methyl-2-butanone was dropwise added. The mixture was stirred for 3 hours at 40 ¤C and the amino nitrile obtained can, if desired without further treatment, be used for the process according to the invention. The amino nitrile may also be isolated by extraction with 50 ml toluene, which can subsequently be distilled off. Extraction obviates the need to remove the inorganic salts, present during the preparation, at the end of the process according to the invention.

Example II

With stirring, simultaneously (0.5 mole) 56 g α-methylvalinonitrile and 130 ml hydrogen peroxide solution (30% = 1.1 mole) were dropwise added to 0.4 l ammonia (12N). The solution was cooled such that the temperature did not exceed 30 ¤C. After 2 hours' stirring, 1 g palladium-on-carbon was used to reduce the excess peroxide. The palladium-on-carbon was filtered off and the water. Layer obtained was evaporated at reduced pressure (T ≤ 40 ¤C). Thus, 48.3 g crystalline α-methylvaline amide (75%) was obtained. An identical result could be obtained starting from a 50% solution of α-methylvalino nitrile in toluene.

Examples III-VIII

In the same way as in Examples I and II, α-methyl-α-amino acid amides and α-cycloalkyl-α-aminoacid amides were obtained. Similar results were obtained if the α-alkyl amino nitriles were added not as such but as a solution in toluene or alcohol. In some cases it is possible to add an α-alkyl-α-amino nitrile to a mixture of ammonia and hydrogen peroxide.
Isolation of the various α-alkyl-α-aminoacid amides was sometimes effected by evaporation, sometimes by crystallization. The results are presented in Table 1. It should be noted that, as the amino nitriles were not first purified, all yields are calculated over two reaction steps, namely the steps described in Examples I and II.

Table 1

| Example | $R_2$ | Amide<br>$R_1$ | Overall yield |
|---|---|---|---|
| III | α-methyl<br>$CH_3$ | leucine amide<br>$(H_3C)_2CH-CH_2-$ | 85 % |
| IV | α-methyl<br>$CH_3$ | butyric acid amide<br>$CH_3-CH_2-$ | 70 % |
| V | α-methyl<br>$CH_3$ | phenylglycine amide<br>$C_6H_5-$ | 75 % |
| VI | α-methyl<br>$CH_3$ | homophenylalanine amide<br>$C_6H_5-CH_2-CH_2-$ | 80 % |
| VII | α-methyl<br>$CH_3$ | (3,4-dimethoxy)phenylalanine amide<br>$3,4-(OCH_3)_2\ C_6H_5-CH_2-$ | 80 % |
| VIII | | 1-carboxylic acid amide-1-amino-<br>cyclohexane-$CH_2-CH_2-CH_2-CH_2-CH_2-$ | 60 % |

## Claims

1. Process for the preparation of α-amino-α-methylcarboxylic acid amides and α-amino-α-cycloalkyl-carboxylic acid amides of the formula

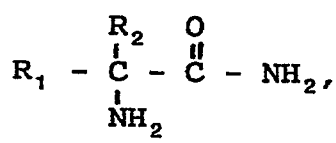

where $R_1$ represents alkyl, alkaryl, aryl, alkenyl, heteroalkyl or heteroaryl, whether or not substituted, and $R_2$ is methyl or forms, together with $R_1$, a cycloalkyl, by conversion of the corresponding amino nitriles using hydrogen peroxide under basic conditions, characterized in that the conversion takes place in the presence of 2–6 moles hydrogen peroxide per mole amino nitrile and in the presence of 3–12 moles ammonia per mole amino nitrile.

2. Process according to claim 1, characterized in that use is made of about 2.2 moles hydrogen peroxide per mole amino nitrile.

3. Process according to claim 1 or 2, characterized in that it is carried out using 5–8 moles ammonia per mole amino nitrile.

4. Process according to any of claims 1–3, characterized in that it is carried out at a temperature between 0 and 50°C.

5. Process according to claim 4, characterized in that it is carried out at a temperature between 25 and 45°C.

## Patentansprüche

1. Verfahren zur Herstellung von α-Amino-α-methylcarbonsäureamiden und α-Amino-α-cycloalkylcarbonsäureamiden der Formel

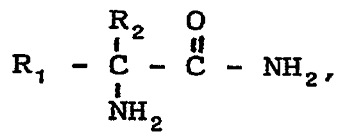

worin $R_1$ Alkyl, Alkaryl, Aryl, Alkenyl, Heteroalkyl oder Heteroaryl, egal ob substituiert oder nicht, bedeutet und $R_2$ Methyl darstellt oder zusammen mit $R_1$ Cycloalkyl bildet, durch Reaktion der peroxid unter basischen Bedingungen, dadurch gekennzeichnet, daß die Reaktion in Anwesenheit von 2 bis 6 Molen Wasserstoffperoxid pro Mol Aminonitril und in Anwesenheit von 3 bis 12 Molen Ammoniak pro Mol Aminonitril durchgeführt wird.

2. Verfahren nach anspruch 1, dadurch gekennzeichnet, daß etwa 2,2 Mole Wasserstoffperoxid pro Mol Aminonitril verwendet werden.

3. Verfahren nach Anspruch 1 oder 2, dadurch gekennzeichnet, daß es es unter Verwendung von 5 bis 8 Molen Ammoniak pro Mol Aminonitril durchgeführt wird.

4. Verfahren nach einem der Ansprüche 1 bis 3, dadurch gekennzeichnet, daß es bei einer Temperatur von 0 bis 50°C durchgeführt wird.

5. Verfahren nach Anspruch 4, dadurch gekennzeichnet, daß es bei einer Temperatur von 25 bis 45°C durchgeführt wird.

## Revendications

1. Procédé pour la préparation d'amides d'acides α-amino-α-méthyl-carboxyliques et d'amides d'acides α-amino-α-cycloalkyl-carboxyliques présentant la formule:

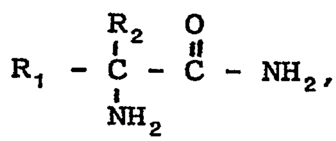

dans laquelle $R_1$ représente un alkyle, un alkaryle, un aryle, un alcényle, un hétéroalkyle ou un hétéroaryle, qu'il soit ou non substitué, et $R_2$ est du méthyle ou bien forme, conjointement avec $R_1$, un cycloalkyle, par conversion des nitriles aminés correspondant en utilisant du peroxyde d'hydrogène sous des conditions basiques, caractérisé en ce que la conversion s'effectue en présence de 2 à 6 moles de peroxyde d'hydrogène par mole de nitrile aminé et en présence de 3 à 12 moles d'ammoniac par mole de nitrile aminé.

2. Procédé selon la revendication la revendication 1, caractérisé en ce que l'on utilise d'environ 2,2 moles de peroxyde d'hydrogène par mole de nitrile aminé.

3. Procédé selon la revendication 1 ou 2, caractérisé en ce qu'il est mis en œuvre en utilisant 5 à 8 moles d'ammoniac par mole de nitrile aminé.

4. Procédé selon l'une quelconque des revendications 1 à 3, caractérisé en ce qu'il est mis en œuvre à une température entre 0 et 50°C.

5. Procédé selon la revendication 4, caractérisé en ce qu'il est mis en œuvre à une température entre 25 et 45°C.